# EUROPEAN PATENT APPLICATION

(11) **EP 0 904 784 A1**
(43) Date of publication of application: **31.03.1999**
(21) Application number: 97202900.3
(22) Date of filing: 22.09.1997
(51) Int. Cl.: A61K 35/74, A23L 1/03

(54) **Probiotic nutritional preparation**

(71) Applicant: N.V. Nutricia, 2700 MA Zoetermeer (NL)
(72) Inventor: Van Hoey-De-Boer, Klaske Anna, 3443 BD Woerden (NL); Hageman, Robert Johan Joseph, 2742 EV Waddinxveen (NL)
(74) Representative: de Bruijn, Leendert C.

(57) **Abstract**

The invention relates to a nutritional preparation with health promoting action, in particular with respect to the prevention and treatment of disorders of the gastrointestinal tract, comprising 10⁶-10¹⁴, preferably 10⁷-10¹³ viable cells, per gram of the total preparation, of of each of the following micro-organisms:
- Bifidobacterium;
- Enterococcus faecium; and
- a Lactobacillus strain that produces predominantly dextro-rotary lactate.

The nutritional preparation can further comprise a corresponding amount of cells of a Lactococcus strain or a Micrococcus strain.

Also, the preparafion preferably contains prebiotic compounds, as well as substances that inhibit bacterial adhesion to the wall of the gastrointestinal tract.

The preparation can be in the form of a food supplement, a ready-to-use food composition, an infant formula or a tube feeding.

## Description

The present invention relates to a nutritional preparation with health promoting action, in particular with respect to the prevention and treatment of disorders of the gastrointestinal tract.

More specifically, the invention relates to such a preparation which contains a combination of the following three types of probiotic micro-organisms:
- a Bifidobacterium-strain;
- Enterococcus faecium, and
- a Lactobacillus strain that predominantly produces dextro-rotary lactate.

In particular, the invention relates to such a preparation in the form of a food supplement or in the form of a food which is ready for consumption.

It is known that certain bacteria, in particular bacteria isolated from the gastrointestinal tract of healthy human beings or animals, as well as certain lactic acid bacteria such as Lactobacilli, have a prophylactic and therapeutic effect on gastrointestinal diseases, such as gastrointestinal infections.

It is also known for this purpose to administer preparations containing these (living) micro-organisms to humans or animals. Following administration, these probiotic (also called eubiotic) bacteria compete with pathogenic bacteria for nutrients and/or attachment sites on the gastrointestinal wall, thereby reducing their numbers and reducing or preventing infections.

The following publications all describe micro-organisms which can be used in the treatment of gastro-intestinal disorders: WO 97/00688; WO 97/20577; WO 89/11858; WO 95/07090; EP 0 391 416; WO 95/33046; EP 0 199 535.

One of the problems in treating gastrointestinal infections is that it can not be ascertained from the obvious symptomes (such as diarrhoea, bloating or cramping), which specific micro-organism is responsable for the disorder. Also, the different harmful micro-organisms (such as enterotoxigenic E.coli strains, rotavirusses, Clostridia, Salmonella or Campylobacter species) preferably reside in, and thereby cause disorder of, different parts of the gastrointestinal tract.

As the bacteria which can be administered for treating the gastrointestinal disorders will also have preferred sites in the gastrointestinal tract for adhering and/or growing, which can differ from the sites where the harmful micro-organisms to be controlled will grow, administering certain types of probiotic bacteria will not help against certain types of gastrointestinal infections. For instance, administration of Lactobacillus, Lactococcus or Microcroccus species, which only grow in the presence of oxygen, will not be helpful against infections in parts of the gastrointestinal tract in which there are essentially anaerobic conditions, such as the colon. However, these are the parts of the gastrointestinal tract where pathogenic micro-organisms such as E.coli, Salmonella, Clostridia, Shigella and Campylobacter preferably reside. On the other hand, bifidobacteria and enterococci are species that grow in the anaerobic part of the GI-tract and will not be active against infections by Staphylococcus or rotavirusses, which colonize the upper part of the GI-tract.

Therefore, treatment with preparations containing only one micro-organism, or one type of micro-organism, will require that the type of infection is determined before suitable treatment can be established, which -due to the necessity of collecting and cultivating the pathogenic micro-organism- can take too much time. Also, this is not practical for household use, as it requires the intervention of skilled medical personel.

It will be clear that the above also holds true for prophylactic treatment: using only one strain or type of bacteria will only protect some parts of the gastrointestinal tract, and therefore only protect against infection of only certain types of micro-organisms.

Purpose of the present invention is therefore to provide a micro-organism containing nutritional preparation, which can be used for treatment of gastro-intestinal disorders, without the need of determining beforehand which micro-organism is responsible for the disorder; and/or which can be used for prophylactic treatment of the entire gastrointestinal tract. In this way, also a commercial preparation suited for household use can be provided.

The present invention therefore relates to a nutritional preparation comprising viable cells of the following probiotic strains:
- Bifidobacterium, and
- Enterococcus faecium, and
- a Lactobacillus strain that produces dextro-rotary lactate,
each of these bacteria being present in an amount of 10⁶-10¹⁴, preferably 10⁷-10¹³ viable cells/gram of the total preparation.

As discussed below, these micro-organisms are chosen because they can pass the stomach; after passage of the stomach and the small intestine, more than 5%, preferably more than 10%, of the administered cells will remain viable, even without encapsulation.

As the Bifidobacterium strain, any strain suited for, and preferably approved for, administration to animals and/or humans can be used, such as B. infantis, B. bifidum, B. adolesentis, B. longum, B. thermophilum, B. globosum, B. lactis or B. catenulatum, or a combination thereof. B. infantis, in particular B. infantis DSM 20088 (also called B.lactis subsp.novum); B. globosum and B. lactis and strains thereof are preferred.

Enterococcus faecium strains suitable for use in the invention comprise (non-limiting) SF 68, PR 88 and M 74, which are all vancomycin-resistant, as well as E. faecium ATCC 19434.

The Lactobacillus strain used is preferably such that it predominantly, more preferably exclusively produces dextro-rotary (L+) lactate. By this is meant that of the lactate produced, less than 5%, preferably less than 2% is levo-rotary lactate. Of course, the micro-organism can produce other metabolites besides the L(+) lactate.

The use of these Lactobacillus strain is preferred because in certain disorders of the GI-tract, such as short bowel syndrome, carbohydrate malabsorption or in cases of carbohydrate overload, levo-rotary lactate will not be disgested (sufficiently), which can cause adverse reactions such as acidosis.

Examples are Lactobacillus rhamnosus (previously called Lactobacillus casei sbsp rhamnosus), such as the preferred strains ATCC 7469 and 271 DSM 6549; Lactobacillus [GG]; L. casei NCIMB 8823 and L. casei DSM 20244.

Other suitable strains of the above micro-organisms are mentioned in the prior art cited above, which is incorporated herein by reference.

As further discussed below, these micro-organisms, as well as any other micro-organisms used, preferably have good adhesion properties to the gastrointestinal wall, in particular mucous membranes thereof and/or any receptors thereon. Also, the bacteria used will preferably by themselves have no adverse effect on the health of the person or animal to be treated. As mentioned, this can be achieved by using micro-organisms which are endogenous to the gastro-intestinal tract (or strains derived therefrom) or micro-organisms which are present in food products, such as lactic acid bacteria.

These micro-organisms can be obtained in any manner known per se, such as cultivating them in suitable media, preferably in such a way and using such media that the microbial preparations thus obtained are suitable for adminstration to humans and/or animals, and especially are able to colonize the desired parts of the gastrointestinal tract.

The bacteria are preferably used in amounts (relating to the total number of bacteria administered) of 0,1-20 parts Bifidobacterium to 0,1-20 parts Enterococcus faecium to 0,1-20 parts Lactobacillus, more preferably in about equal amounts.

These bacteria are preferably each administered in an amount of 10⁷ -10¹³ cells/day, preferably 10¹⁰ - 10¹¹ cells/day, in 1 to up to 10 doses/day, both for adults as well as children. The nutritional composition of the invention, in particular the amounts of micro-organisms therein per unit of volume or weight, is preferably such that such a daily dosis can easily be provided, as well as administered.

Besides the three types of micro-organisms mentioned above, the preparations of the invention can contain one or more further micro-organisms which prevent the growth and/or the adherence of pathogenic microoganisms in the gastrointestinal tract. Specific examples are non-pathogenic E.coli strains, in particular E.coli strains that do not produce any toxins and do not contain any plasmids.

Also very suitable are Lactobacillus strains (including those that do not predominantly produce dextro-rotary lactate), such as Lactobacillus GG, of which strain ATCC 53103 is preferred, as well as other lactic acid bacteria, for instance Lactococcus species/strains, such as Lactococcus lactis, of which strain LW-P53 is preferred.

Instead of, or in combination with, Lactococcus strains, Micrococcus strains can also be used, such as M. luteus (of which M. Luteus ATCC 4698 is particularly preferred), M. varians, M. kristinae or M. sedentarius.

Other suitable strains of micro-organisms are mentioned in the prior art cited above, which is incorporated herein by reference.

These further micro-organisms can also be present in the preparations of the invention in amounts of 10⁷ - 10¹³, preferably 10⁸-10¹², more preferably 10¹⁰ - 10¹¹ viable cells/gram of the total preparation, most preferably in amounts in the range of the three micro-organisms mentioned above. They are also administered in amounts/doses as mentioned above.

The preparation of the invention is preferably in the form of a food supplement, or in the form of a food or food composition which as such is ready for consumption.

When the preparation of the invention is in the form of a food supplement, it can be in a form for separate administration, such as a capsule, a tablet, a powder or a similar form, containing preferably a unit dose of the micro-organisms, containing 10⁷-10¹³ cells/dose, preferably 10¹⁰ - 10¹¹ cells/dose.

The food supplement can also be in the form of a powder or a similar form, which is added to, or mixed with, a suitable food (composition) or a suitable liquid or solid carrier, for the preparation of a food which is ready for consumption.

A preferred embodiment is a freeze-dried powder of the micro-organisms, which can be in the form of a sachet, or which can be incorporated in a capsule or a tablet or another dry administration form. These freeze-dried preparations can be obtained using suitable techniques and can contain suitable adjuvants known per se, for instance cryoprotectants such as maltose.

For instance, the invention in the form of a food supplement can be in the form of a freeze-dried powder, which is reconstituted using a suitable liquid, such as water, oral rehydration solution, milk, fruit juice, or similar drinkable liquids. It can also be in the form of a powder which is mixed with solid foods, or foods with a high water-content, such as fermented milk products, for example yoghurt.

The nutritional preparations of the invention can also be in the form of a food which is ready for consumption. Such a food can for instance be prepared by
- adding a supplement of the invention as described above to a food or food base known per se;
- adding the micro-organisms (seperately or as a mixture) in the amounts required for administation to a food or food base known per se; or
- by cultivating the required bacteria in a food medium until a food containing the amount of bacteria required for administration is obtained. This medium is preferably such that it already forms part of the food, or will form part pf the food after fermentation.

In this respect, the nutritional preparation can be either fermented or non-fermented.

The nutritional preparations in the invention can be foods for oral consumption, for instance a total food; an infant formula; or a food for administration by tube or cathether into the stomach or the gut.

As the probiotics-containing preparations of the invention also stimulate the immune system, they can also be used as or in a clinical feeding or food preparation for patients suffering from chronic infections. They are also useful as a food or supplement to prevent or treat allergies, such as occur in IBS or cow's milk allergy.

The nutritional preparation of the invention can further comprise all desired components, and/or additives which are suited for use in food or food supplements, including flavors, colorings, preservatives, sugar etc, as long as they do not affect the viablity of the micro-organisms present therein. Some preferred specific additives are discussed hereinbelow.

The preparation can further contain prebiotic compounds, in particular specific fibers that produce butyrate/butyric acid or propionat/propionic acid upon fermentation; nitrogen donors such as proteins; and specific vitamins, minerals and/or trace elements. With respect to the latter, and as can be seen from the examples, the presence of increased or moderately high amounts of vitamin A, K, B12, biotine, Mg and Zn can be advantageous, as can the presence of folic acid in preparations intended for the treatment of chronic diarrhoea.

The fibres can be used in an amount of at least 0,5 g fibre per 100 g of the total preparation. When formulated as a food for tube feeding, the composition of the invention preferably contains 0,01 - 1,5 g specific fibres per 10¹⁰ micro-organisms present in the composition; a dry supplement according to the invention preferably contains 1 - 100 g specific fibres per 10¹⁰ micro-organisms present in the composition.

Protein can be present in amounts of 0,1 mg to 4 g, both in a food for tube feeding as well as in a dry supplement.

As the fibres, the preparation preferably contains a resistant starch or another butyrate generator, as well as a suitable propionate generator such as gums or soy polysaccharides, in the amounts indicated above. Short chain fatty acids such as butyric acid and propionic acid can also be used as such, preferably in a suitably encapsulated form, or as a fysiological equivalent thereof, such as sodium propionate, in an amount of at least 0,1 g per 100 g of the total composition.

As a source of nitrogen, vitamins, minerals and trace elements, yeast extract can be used, in amounts of 10 mg to 2 g per 10¹⁰ micro-organisms present in the composition, and/or in an amount of at least 0,5 g per 100g of the total preparation.

The nutritional composition can also contain growth factors such as those present in milk whey or colostrum.

The preparation of the invention can further contain one or more substances that inhibit bacterial adhesion to the epithelial wall of the gastrointestinal tract. Preferably, these compounds are selected from lectins, glycoproteins, mannans, glucans, chitosan and/or derivatives thereof, charged proteins, charged carbohydrates, sialylated compounds and/or adhesion-inhibiting immunoglobulins, as well as modified carbohydrates and modified chitin, the latter in amounts of 1-10 % w/v, preferably 2-5 % w/v of the composition.

Very suitable adherence-inhibiting substances are chitosan, carob flower, as well as extracts which are rich in condensed tannin and tannin-derivatives, such as cranberry extract; the amount of tannin in the final product preferably being 10-600 µg/ml.

It is very surprising that, while these compounds prevent and/or reduce the adhesion of harmful micro-organisms, they do not appear to significantly affect the health promoting action of the probiotic micro-organisms administered according to the invention.

The preparations of the invention can also contain antibodies such as immunoglobulins that act specifically against the pathogenic micro-organisms, more specifically against enterotoxigenic E.coli strains, rotavirusses, Helicobacter, Clostridia, Salmonella and/or Campylobacter species. These immunoglobulins are used in amounts of at least 20 µg/100 g of the composition. The compositions can also contain a bactericidal compound, such as preferably lactoferrin or lysozyme, in an amount of at least 2 mg/100 g product. These can also be combined with protease inhibitors.

The preparation, in particular when in the form of a total food, can also contain peptides and/or proteins, in particular proteins that are rich in glutamate and glutamine, lipids, carbohydrates, vitamins, minerals and trace elements. The use of glutamine/glutamate precursors, in amounts corresponding to 0,6-3 g glutamine/100g product, as well as of small polypeptides that contain a high amount of glutamines, is preferred. Alternatively, proteins that are rich in glutamine, such as milk proteins, wheat proteins or hydrolysates thereof, can be added.

As additives, preferably at least one proprionate generator, at least one butyrate generator and at least one glutamine/glutamate-precursor are present. This can be a fibre, such as the resistant starches mentioned above; a protein; or a seperate precursor compound known per se.

The preparations of the invention are preferably lactose-free, and do not have a high osmolality (preferably less than 400 mosm/l, more preferably less than 300 mosm/l).

All micro-organisms used in the invention are preferably resistant to degrading conditions in the gastrointestinal tract, such as amylase, stomach acids, bile (salts), lipases and/or pancreatic fluid, so that they can pass the stomach and have their beneficial influence on the gastrointestinal tract. Therefore, they can be used as such without precaution as to their passing the stomach. However, preferably the oxygen-sensitive Bifidobacteria are encapsulated for passage of the stomach by means of a suited delayed release encapsulation. Suitable compounds for encapsulation are chitosan, maltodextrin, dextrins, lipids, polylactate, and poly- or oligosaccharides.

According to a specifically preferred embodiment, all micro-organisms used are encapsulated, when used in liquid or moist products. This prevents that the micro-organisms, which are present present in the preparations of the invention as living or at least viable cells, start growing in and/or fermenting the food product, thereby reducing its shelf-life. This is a problem with almost all ready-to-use type preparations of the invention, in particular when a food or food base is used that provides suitable conditions for bacterial growth, such as an aqueous medium and sources of nutrients for the micro-organisms ( which will be the case with almost all consumption foods). It can however also be a problem with nutritional supplements for household use, which are added to food products, and then kept for some time, as the bacteria of the invention can appreciably start fermenting and/or degrading any suitable food medium within half an hour. For instance, when the preparations of the invention are combined with food products on the basis of cereals, in particular when these have a high water content, the shelf life is drastically reduced.

In view of this, encapsulation of the micro-organisms can provide an improved shelf-life of two years or more, in particular for food products which are ready for consumption. They also improve the safety of supplements for adding to food products prior to consumption. Also, such encapsulation can even further improve the resistance against stomach fluids and/or pancreatic fluid.

Suitable compounds for encapsulation for improving the shelf life, as well as methods for carrying out the encapsulation, are known in the art. Besides the compounds mentioned above, preferred compounds include combinations of chitosan and maltodextrine, and the like.

Although the invention is not limited to any specific explanation, it is assumed that the probiotic micro-organisms produce their prophylactic and therapeutic effect by competing for nutrients etc., and in general providing surroundings in the gastrointestinal tract which are not suited for the growth of the pathogens, or can even kill them.

The probiotic micro-organisms also adhere to the walls of the gastrointestinal tract, in particular with the mucous membranes and/or receptors thereon, and thereby compete with the pathogenic micro-organisms under the dynamic conditions present in the GI-tract. This will prevent that the pathogens infect cells, and will also speed up clearance of the pathogens from the gastrointestinal tract. This prevention of adherence (by both living and dead pathogens, as well as fragments thereof) can be increased considerably by including anti-adhesive agents in the compositions. By including specific fibres, the beneficial micro-organisms will have sufficient substrate available for growth and produce factors that can heal the damaged gut tissue.

The preparations of the invention will in general have a positive influence on the gastrointestinal tract. For this purpose, they can further contain health improving compounds known per se, such as medicaments etc. In particular, the preparations can contain compounds which have a beneficial influence on the gastro-intestinal tract, such as glutamine/glutamate or precursors thereof.

The preparations of the invention can be used for preventing or treating gastrointestinal disorders, such as gastrointestinal infections, diarrhoea, systemic infections, or disturbance in the immune system, in particular those caused by pathogens such as such as enterotoxigenic E.coli strains, rotavirusses, Clostridia, Salmonella or Campylobacter species.

Also, an insufficient level of probiotic micro-organisms in the gastrointestinal tract can lead to an increased permeability of the gastrointestinal wall, which can increase the immunogenic and allergenic properties of food components or other orally administered compounds. The preparations of the invention can also be used to treat or prevent these phenomena.

Also, as is known, treatment with antibiotics, in particular oral antibiotics, can disturb or destroy the gastrointestinal flora. The preparations of the invention can either be used to prevent this (usually by administration in combination with the antibiotics); for preventing the colonisation by harmful organisms after treatment is ended; or for restoring the gastrointestinal flora after antibiotic treatment.

The main advantage of the preparation of the invention is that the different micro-organisms used colonise and grow in different parts of the gastrointestinal tract, and therefore provide overall treatment and/or protection of the gastrointestinal tract. For instance, Lactobacillus, Lactococcus or Micrococcus species only grow in the presence of oxygen, whereas Bifidobacteria and enterococci prefer anaerobic conditions. Lactobacilli, lactococci and micrococci can already settle in the mouth, and preferably settle in the small intestine, in particular up to the ileum. Enterococcus species preferably settle in the ileum and the colon. Bifidobacteria preferably settle in the colon. Also, rod-shaped lactobacilli and round lactococci also settle in different surroundings, thereby supplementing each others action. In this way, a synergistic effect can be obtained.

The preparations of the invention further can be used in the therapy or profylaxis of all kinds of other disorders of the gastrointestinal tract, such as IBS, Crohn's disease, cancer of the GI tract, impaired immune function against bacteria, fungi (such as Candida albicans), yeasts and/or virusses, obstipation, diarrhea as observed during travelling, antibiotics therapy or radiotherapy, diarrhea in low birthweight children, disorders of the GI-tract as may occur in cystic fibrosis, pancreas diseases, liver diseases and several kidney diseases and in food borne allergies (such as coeliac disease, lactose allergy and cow's milk allergy), lactose intolerance and similar disorders.

Also, the probiotics-containing preparations can be used to prevent and/or treat disorders of the heart or bloodcirculation. One reason for this is that, due to the presence of the probiotics, the cholesterol levels in the blood are lowered and infections, such as infections of the heart valves, caused by Streptococcus species are reduced. The probiotics can also be effective against acute rhumatics and vaginitis.

The invention will now be illustrated by means of the following non-limiting Examples.

### Example 1: Powder A enriched with probiotics

1. Standard media are used for growing Lactobacilli, Enterococci, Micrococci and Bifidobacter, for example a medium providing 15-20 g peptone or tryptone per litre and about 5 g yeast extract per litre. For preparing the media the standard procedure is applied (concentration of components, sterilization, inoculation) and the microorganism is allowed to grow at elevated temperature (e.g. 35-42°C) for a sufficient time (e.g. 8-48 hours).
   In this way separate cultures are obtained of Lactobacillus rhamnosus 271 DSM 6594, Enterococcus faecium ATCC 19434, Bifidobacter infantis DSM 20088 and Micrococcus ATCC 4698.
2. Aliquots are taken and mixed together.
3. The mixture is sprayed over a fluid bed of maltodextrin DE 32 of a particular sieve size at elevated temperature. A free flowing powder results, which contains a count of viable cells between 10¹⁰- 10¹² per g and shows a shelf life of several weeks.

### Example 2: Powder B enriched with probiotics

The mixture that is obtained in step 2 of example 1 is blended with maltodextrine to a dry matter content of 70% and free dried. The powder contains 10'10-10'11 viable cells of each of the microorganisms per g.

### Example 3: Capsule for daily use for the prevention of gastrointestinal disorders

The capsules consist of about 3 g of an emulsion that comprises:
- 1 g probiotics powder B comprising Lactobacillus rhamnosus, Entercoccus feacium, Bifidobacter infantis, Lactococcus lactis and Micrococcus.
- 1 g novelose and 0.4 g arabic gum,
- 0.4 g marine oil, comprising 0.2 g sodium propionate
and a capsule wall of chitosan.

### Example 4: Sachet with powder for supplementing regular food or drinks

Two hundred kilo's of the powder of example 2 is dry mixed with
400 kg glucose
70 kg NaCl
22 kg KCl
50 kg Potassium citrate monobasic.

The mixture can be used in the preparation of oral rehydration solutions or be packed in sachets comprising 7.5 g of the powder. One sachet can be reconstituted by the consumer in 200 ml (boiled) water or added to their dessert or other food component.

### Example 5: Sachet comprising probiotics, prebiotics, antiadhesives, minerals and vitamins

The following ingredients are mixed together:
- 10 kg of a spraydried whey extract that is rich in immunoglobulins. The extract is obtained by precipitating the caseins from defatted milk and isolating the basic fraction by chromatography, including some important growth factors immunoglobulins and lactoferrin. The milk can be colostrum or derived from hyperimmunized animals.
- Vitamins and minerals as pure ingredient or premix, providing:
   800 mg vitamin K
   5 g biotin
   100 mg cyanocobalamine
   15 g folic acid
   50 g RE vitamin A
   100 g pantothenic acid
   500 g ascorbic acid
   20 kg magnesium phosphate monobasic
   40 kg magnesium chloride
   3 kg zinc citrate
- 10 kg spraydried cranberry extract
- 170 kg Novelose and 40 kg Soy polysaccharide.

This mixture is packed in sachets of about 2.9 g which are used together with the sachets from example 4.

Two hundred ninety (290) kg of the mixture can also be mixed with 750 kg of the mixture of example 4 which is packed in sachets of about 10.5 g, which can be used by persons as described in example 4.

### Example 6: Fermented complete food

By methods that are known in the art a complete liquid formule is composed based on caseinats, vegetable oils, maltodextrin and vitamin/mineral premixes.

Two thousand (2000) litres of this liquid composition is fermented during 4 hours at 35-42°C by inoculating with a sufficient amount of the cultures as described in example 1. The fermented product is pasteurized and packed aseptically in cartons.

### Example 7: Infant formula with probiotics

To 1000 kg of a spraydried infant formula is dry blended 10 kg of the mixture as obtained in example 2 and packed in tins or cartons as known in the art.

### Example 8: Tube feeding with probiotics

The microorganisms as described in step 2 of example 1 are encapsulated by methods known in the art to become sustained released, for example by including them in small particles coated with a fibre type constituent or chitin, which dissolves slowly in the stomach but mostly in the lower parts of the gut.

To a regular tube feeding is added after pasteurization a proportional amount of an aqueous pasteurized suspension of encapsulated probiotics to obtain a final concentration of about 10⁸ viable cells per ml.

## Claims

1. Nutritional preparation with health promoting action, in particular with respect to the prevention and treatment of disorders of the gastrointestinal tract, comprising 10⁶-10¹⁴, preferably 10⁷-10¹³ viable cells, per gram of the total preparation, of each of the following micro-organisms:
- Bididobacterium;
- Entercoccus faecium; and
- a Lactobacillus strain that produces predominantly dextro-rotary lactate.

2. Nutritional preparation according to claim 1, comprising
- Bifodobacterium infantis, Bifodobacterium globosum or Bifodobacterium lactis;
- Enterococcus faecium, preferably strain SF 68
- Lactobacillus rhamnosus, preferably strain ATCC 7469.

3. Nutritional preparation according to claim 1 and/or 2, further comprising 10⁶-10¹⁴, preferably 10⁷-10¹³ viable cells, per gram of the total preparation, of a Lactococcus strain, preferably of Lactococcus lactis; or of a Micrococcus strain, preferably M. luteus.

4. Nutritional preparation according to any of the preceding claims, further comprising a prebiotic compound, preferably chosen from fibres that produce butyrate/butyric acid or propionate/propionic acid upon fermentation, such as resistant starch, gums, soy polysaccharides and/or proteins.

5. Nutritional preparation according any of the preceding claims, comprising at least 0,5 g fibre and/or at least 0,5 g yeast extract per 100 g of the total preparation.

6. Nutritional preparation according to any of the preceding claims, further comprising one or more substances that inhibits bacterial adhesion to the epithelial wall of the gastrointestinal tract, preferably chosen from the group lectins, glycoproteins, mannans, glucans, chitosan and/or derivatives thereof, charged proteins and/or adhesion-inhibiting immunoglobulins, modified carbohydrates and modified chitin, carob flower, as well as extracts which are rich in condensed tannin and tannin-derivatives, such as cranberry extract.

7. Nutritional preparation according to any of the preceding claims, further comprising
- antibodies, such as immunoglobulins, that act specifically against the pathogenic micro-organisms, more specifically against enterotoxigenic E.coli strains, rotavirusses, Clostridia, Salmonella and/or Campylobacter species, and preferably in amounts of at least 20 µg/100 g of the total preparation;
- sialylated compounds, preferably in amounts of at least 2 mg/100 g of the total preparation; and/or
- a bactericidal compound, such as lactoferrin, preferably in amounts of at least 2 mg/100 g of the total preparation.
- short chain fatty acids, preferably in a suitably encapsulated form, or a fysiological equivalent thereof, such as sodium propionate, in an amount of at least 0,1 g per 100 g of the total composition.

8. Nutritional preparation according to any of the preceding claims, further comprising at least one further micro-organism which prevents the growth and/or the adherence of pathogenic micro-oganisms in the gastrointestinal tract, preferably chosen from
- non-pathogenic E.coli strains, in particular E.coli strains that do not show profasing and do not produce any toxins, and/or do not contain any plasmids.
- Lactobacillus GG, as well as other lactic acid bacteria.

9. Nutritional preparation according to any of the preceding claims, in which the different strains of micro-organisms are present in about equal amounts.

10. Nutritional preparation according to any of the preceding claims, wherein at least the Bifidobacterium cells, but preferably all bacterial cells, are encapsulated in such a way that the cells are predominantly released in the gut.

11. Nutritional preparation according to any of the preceding claims, wherein at least the bacterial cells are encapsulated so as to provide improved life for food compositions containing these cells.

12. Nutritional preparation according to any of the preceding claims, further comprising proteins and/or peptides, in particular proteins and/or peptides that are rich is glutamine/glutamate; lipids; carbohydrates; vitamins; minerals and/or trace elements.

13. Nutritional preparation according to any of the preceding claims, in the form of a food supplement, preferably comprising a freeze-dried preparation of the micro-organisms used.

14. Nutritional preparation according to any of the preceding claims, in the form of a food composition which is ready for consumption, preferably obtained by either
- mixing the micro-organisms with a suitable food or food base;
- cultivating the micro-organisms in a suitable food or food base; or
- adding a supplement according to claim 13 to a suitable food or food base.
